Europäisches Patentamt

**European Patent Office**

Office européen des brevets

(11) Numéro de publication: **0 040 572**
**B1**

(12) **FASCICULE DE BREVET EUROPEEN**

(45) Date de publication du fascicule du brevet:
27.07.83

(21) Numéro de dépôt: 81400782.9

(22) Date de dépôt: 19.05.81

(51) Int. Cl.³: **A 61 K 39/002**

(54) Procédé d'obtention de préparations de toxoplasmes pour le diagnostic de la toxoplasmose, et préparations ainsi obtenues.

(30) Priorité: **20.05.80 FR 8011261**

(43) Date de publication de la demande:
**25.11.81 Bulletin 81/47**

(45) Mention de la délivrance du brevet:
**27.07.83 Bulletin 83/30**

(84) Etats contractants désignés:
**AT CH DE GB LI NL SE**

(56) Documents cités:
**FR-A-2 226 468**
**ANNALES DE BIOLOGIE CLINIQUE, vol. 28, 1970, PARIS (FR) P. COUZINEAU et al.: »Agglutination directe des toxoplasmes; Préparation de l'antigène et examen de 400 sérums«, pages 411—415**

(73) Titulaire: **BIOMERIEUX, Société Anonyme dite, Marcy l'Etoile, F-69260 Charbonnieres les Bains (FR)**

(72) Inventeur: **Trouyez, Gérard, Marcy l'Etoile, F-69260 Charbonnieres les Bains (FR)**

(74) Mandataire: **Hufénus, Christiane et al, CABINET BEAU DE LOMENIE 55, rue d'Amsterdam, F-75008 Paris (FR)**

## Procédé d'obtention de préparations de toxoplasmes pour le diagnostic de la toxoplasmose, et préparations ainsi obtenues

La présente invention a trait à un procédé d'obtention de préparations de toxoplasmes, c'est-à-dire de suspensions de toxoplasmes destinées au diagnostic de la toxoplasmose par agglutination directe.

Le diagnostic d'infection aiguë avec des toxoplasmes Gondii dépend des résultats du test sérologique. Nombre de problèmes liés aux méthodes disponibles de sérodiagnostic de cette infection ont été un stimulant pour la recherche d'autres méthodes. Actuellement, les méthodes courantes dont les biologistes disposent en pratique sont d'un coût élevé à réaliser, longues ou d'une sensibilité insuffisante pour être utiles lors du premier stade de l'infection [cf. test d'hémagglutination: Ann. Biol. Clin. 28, 411 – 415 (1970)].

La méthode proposée permet à la fois d'obtenir des antigènes de toxoplasmes en grande quantité et d'améliorer très largement la fidélité et la sensibilité du test d'agglutinations, en supprimant notamment les agglutinations non spécifiques. Les méthodes employées couramment jusqu'à présent utilisaient des organismes entiers et morts. Ces méthodes très simples ont cependant deux caractéristiques défavorables:

- Tout d'abord, elles manquent de sensibilité. Dans le test d'agglutination, le titre est généralement beaucoup plus bas que dans le test de lyse ou dans le test traditionnel d'immunofluorescence. Par voie de conséquence, quelques sérums qui peuvent être positifs dans les deux derniers tests sont en fait présentés comme négatifs par la méthode d'agglutination.
- D'autre part, elles manquent de spécificité. Quelques sérums, qui sont négatifs dans les tests de lyse et d'immunofluorescence, donnent un résultat positif au test d'agglutination.

L'invention se propose de décrire une méthode relative à la préparation d'un antigène qui accroît la sensibilité du test d'agglutination, et supprime les agglutinations non spécifiques. Le test d'agglutination ainsi modifié permet d'obtenir des résultats tout à fait équivalents à ceux obtenus par les tests de lyse.

La technique et la lecture de la nouvelle méthode proposée sont si simples et précises que la mise à disposition d'un tel antigène rendra des services considérables aux laboratoires qui pratiquent la sérologie, que ce soit occasionnellement ou à une échelle beaucoup plus importante.

L'invention a pour objet un perfectionnement du procédé de préparation d'un antigène résultant de la culture des toxoplasmes par injection simultanée à des souris, par voie intra-péritonéale, de toxoplasmes et de cellules sarcomateuses, telles que les cellules du sarcome TG 180 (FR-A-2 226 468). Le procédé selon l'invention permet d'obtenir un nombre considérable de toxoplasmes par souris, équivalent à environ dix fois ce qui pourrait être obtenu par les méthodes conventionnelles, selon lesquelles seul le toxoplasme était inoculé aux souris.

L'invention a pour objet un procédé de fabrication d'une préparation d'antigènes de toxoplasmes selon lequel on inocule par voie intra-péritonéale à des souris des mélanges de toxoplasmes et de cellules sarcomateuses, telles que les cellules du sarcome TG 180, et on récolte les toxoplasmes dans le liquide d'ascite obtenu, caractérisé en ce qu'on opère en deux étapes: dans une première étape, on inocule à des souris un mélange de liquide d'ascite contenant des toxoplasmes et de liquide d'ascite contenant des cellules sarcomateuses, et on récolte l'exsudat péritonéal dont on sépare par centrifugation des cellules sarcomateuses infectées par des toxoplasmes; dans une deuxième étape, on inocule à des souris un mélange de cellules sarcomateuses infectées obtenues dans la première étape et de cellules sarcomateuses non infectées, et on récolte, de 48 à 72 h après l'inoculation, l'exsudat péritonéal des souris qu'on soumet à l'action de la trypsine pendant le temps juste nécessaire pour obtenir la rupture des cellules libérant les toxoplasmes.

Les souris inoculées avec un mélange de cellules sarcomateuses et de parasites développent un exsudat contenant à la fois des cellules sarcomateuses et des parasites, dont la proportion varie à la fois en fonction du rapport des parasites aux cellules sarcomateuses inoculées et du temps écoulé après l'inoculation. L'examen au microscope de ces exsudats permet de distinguer six stades:

Dans un premier stade, la plupart des cellules ne sont pas infectées, et les quelques cellules infectées ne contiennent que peu de parasites,

Dans un deuxième stade, 5 à 10% des cellules sont infectées, mais ne contiennent toujours chacune que peu de parasites.

Dans un troisième stade, la moitié environ des cellules sont infectées, la plupart d'entre elles ne contenant que peu de parasites. Un nombre limité de cellules est très fortement infecté. Il y a peu de toxoplasmes extra-cellulaires.

Dans un quatrième stade, presque toutes les cellules sont largement infectées et près de l'éclatement. On trouve de nombreux parasites extra-cellulaires, un nombre infime cependant si on les compare aux très abondants organismes intra-cellulaires.

Dans un cinquième stade, on trouve encore un grand nombre de cellules largement infectées, proches de l'éclatement. Parallèlement, on peut observer un grand nombre de toxoplasmes libres dont la morphologie semble normale.

Dans un sixième stade, enfin, toutes les cellules sarcomateuses ont été disloquées. Il existe un nombre très important de parasites libres. Beaucoup d'organismes sont manifestement morts ou agglutinés.

L'obtention d'un antigène satisfaisant pour le test d'agglutination, aussi bien d'ailleurs que pour les tests d'immunofluorescence ou de coloration, suppose le respect impératif des deux points suivants: les exsudats doivent être récoltés aux quatrième et cinquième stades exclusivement; les souris devront être inoculées moins de 72 h avant la récolte de cet exsudat, le délai optimal étant de 48 h. Ces deux conditions sont difficiles à remplir si les souris sont inoculées avec des cellules sarcomateuses non infectées, mélangées avec des toxoplasmes obtenus à partir de souris qui été inoculées avec des parasites seuls. C'est la raison pour laquelle un procédé en deux stades a été développé.

On conserve la souche RH de toxoplasmes par des passages successifs de souris à souris, intra-péritonéaux, tous les deux ou trois jours. Les cellules sarcomateuses pour leur part font l'objet de passages intra-péritonéaux sur souris tous les 10 à 12 jours.

La préparation de l'antigène selon l'invention suppose que soient réalisées deux sortes de transferts:

Tout d'abord, dans un premier temps, les souris sont inoculées avec un mélange de toxoplasmes et de cellules sarcomateuses. Par exemple, 2 ml d'un exsudat de cellules sarcomateuses évoluant depuis 10 à 12 jours sont mélangés avec tout le liquide ascitique d'une souris inoculée deux à trois jours auparavant avec la souche RH de toxoplasmes. Ce mélange est alors centrifugé et le sédiment est inoculé à des souris par voie intra-péritonéale. Deux jours plus tard, l'exsudat est examiné au microscope pour vérifier l'absence de bactéries et l'état d'infection des cellules sarcomateuses.

Dans un deuxième temps, on mélange les cellules obtenues lors du premier transfert avec un nombre adéquat de cellules sarcomateuses non infectées. A cet effet, on centrifuge, d'une part, l'exsudat des souris inoculées lors du premier transfert (contenant les cellules infectées) et, d'autre part, l'exsudat de souris auxquelles on a inoculé seulement des cellules sarcomateuses (contenant des cellules non infectées) et on mélange les cellules sédimentées.

Le rapport optimal des cellules non infectées aux cellules infectées dans le mélange varie en fonction du stade d'infection des souris du premier transfert, comme le montre le tableau I ci-après.

Un certain volume, par exemple 2/10 ml, du mélange approprié de cellules non infectées et infectées est ensuite inoculé par voie intra-péritonéale à de nouvelles souris. Deux jours plus tard, l'exsudat péritonéal aura normalement atteint les quatrième et cinquième stades, étant alors prêt à être récolté pour la préparation de l'antigène.

L'étape suivante consiste à libérer, par action de la trypsine ou d'un équivalent, les toxoplasmes des cellules sarcomateuses infectées. Les cellules fortement infectées sont facilement détruites par ce traitement. Si la période à la trypsine est trop longue, ou si la concentration de l'enzyme est trop élevée pendant cette exposition, les toxoplasmes auront une valeur plus faible en tant qu'antigène, bien qu'ils conservent leur morphologie. C'est la raison pour laquelle ce processus doit être interrompu par centrifugation et rejet de la trypsine, dès que le plupart des toxoplasmes ont été libérés des cellules sarcomateuses. On opère donc de la façon suivante:

Le sédiment provenant de l'exsudat des souris de la seconde étape (stades IV et V d'infection) est mis en suspension dans une solution saline tamponnée au phosphate (pH 7,2) ou »PBS«, contenant 0,05% de trypsine et incubée au bain-marie à 37°C sous agitation continuelle. Des parties aliquotes sont examinées toutes les cinq minutes et, aussitôt qu'on constate la rupture des cellules, on centrifuge la suspension.

Le sédiment est à nouveau mis en suspension dans une solution de PBS et centrifugé. Après cette seconde centrifugation, les parasites sont mis en suspension dans une solution à 6% de formaldéhyde, dans du PBS. Les parasites sont maintenus toute une nuit dans dans la solution formolée. Le jour suivant (au moins 16 h après la mise en suspension), ils sont centrifugés de nouveau. Le sédiment est alors lavé à plusieurs reprises dans du PBS, de façon à retirer les débris de cellules et le formaldéhyde. Les parasites sont ensuite mis en suspension dans un tampon alcalin (pH 8,7) contenant un agent conservateur.

La concentration de formol est importante. Les parasites devront conserver leur forme normale de croissant. Quand la fixation est insuffisante, on obtient un antigène moins sensible.

Quand la suspension est vérifiée au microscope, on observe généralement qu'elle est presque entièrement constituée de parasites libres. Quand il ne reste que peu de cellules sarcomateuses, elles sont éliminées par centrifugation. S'il y a de la matière fibrineuse et coagulée, celle-ci peut être éliminée par filtration. La concentration de parasites est adaptée pour donner des résultats optimaux lors du test avec des sérums reconnus positifs au test de lyse. La suspension est maintenue à 4°C. La dilution optimale est déterminée par titrage des différentes solutions de la suspension mère, par rapport au standard OMS ou par rapport à un étalon secondaire rapporté au standard OMS. En règle générale, une agglutination maximale de + + + est obtenu jusqu' à avoir une dilution de 1/4000 du standard OMS, qui correspond à une concentration finale de 0,125 unité internationale (U. I.) par millilitre dans le godet de réaction. Les lectures de + +, + sont obtenues avec des dilutions de 1/8000 et 1/16 000 (0,063 et 0,031 UI/ml). Un résultat douteux ou négatif est obtenu avec des dilutions de 1/32 000 (0,016 UI/ml). La dilution de la suspension utilisée comme antigène est celle pour laquelle les résultats sont les plus nets dans cette échelle de valeur. Elle contient, dans la plupart des cas, approximativement de 10 000 à 50 000 et, de préférence, 20 000 organismes par microlitre. Cette concentration en parasites est donc généralement inférieure à celles antérieurement décrites.

Avec cet antigène, la réaction d'agglutination sur les sérums est conduite en plaques de microtitration en présence de 2-mercapto-éthanol, ou équivalent. Les résultats sont exprimés en titre (inverse de la dilution) ou en unités internationales (par rapport à l'étalon OMS). L'antigène ainsi décrit peut également être utilisé en l'absence de 2-mercapto-éthanol.

Tableau I

Rapport optimal des cellules infectées aux cellules non infectées pour le deuxième transfert

| Stade d'infection | Volume des cellules infectées (sédiment non dilué) | Volume des cellules non-infectées (sédiment non dilué) |
|---|---|---|
| I | 1 | 1 |
| II | 1 | 2 |
| III | 1 | 4 |
| IV | 1 | 16 |

## Revendications

1. Procédé de fabrication d'une préparation d'antigènes de toxoplasmes selon lequel on inocule par voie intrapéritonéale à des souris des mélanges de toxoplasmes et de cellules sarcomateuses telles que les cellules du sarcome TG 180, et on récolte les toxoplasmes dans le liquide d'ascite obtenu, caractérisé en ce qu'on opère en deux étapes: dans une première étape, on inocule à des souris un mélange de liquide d'ascite contenant des toxoplasmes et de liquide d'ascite contenant des cellules sarcomateuses, et on récolte l'exsudat péritonéal dont on sépare par centrifugation des cellules sarcomateuses infectées par des toxoplasmes; dans une deuxième étape, on inocule à des souris un mélange de cellules sarcomateuses infectées obtenues dans la première étape et de cellules sarcomateuses non infectées, et on récolte, de 48 à 72 h après l'inoculation, l'exsudat péritonéal des souris qu'on soumet à l'action de la trypsine pendant le temps juste nécessaire pour obtenir la rupture des cellules libérant les toxoplasmes.

2. Procédé selon la revendication 1, caractérisé en ce que, dans le première étape, on inocule à des souris un mélange de liquide d'ascite d'une souris inoculée deux ou trois jours auparavant par des toxoplasmes, et de liquide d'ascite de souris inoculées 10 à 12 jours auparavant avec des cellules sarcomateuses, on récolte l'exsudat des souris inoculées avec le mélange, de quarante-huit à soixante douze heures après l'inoculation.

3. Procédé selon l'une des revendications 1 et 2, caractérisé en ce que, dans la deuxième étape, le rapport du nombre des cellules sarcomateuses infectées à celui des cellules sarcomateuses non infectées varie en fonction du stade d'infection des souris dans la première étape.

4. Procédé selon l'une des revendications 1 à 3, caractérisé en ce que le sédiment de l'exsudat récolté lors de la seconde étape est exposé au bain-marie à 37°C à l'action d'une solution à 0,05% de trypsine dans une solution saline tamponnée à pH 7,2.

5. Procédé selon la revendication 4, caractérisé en ce que les toxoplasmes libérés sont, après sédimentation, remis en suspension dans une solution à 6% de formol, pendant au moins 16 heures, puis centrifugés, lavés et mis en suspension dans un tampon alcalin à pH 8,7 contenant un agent conservateur.

6. Procédé selon la revendication 5, caractérisé en ce que la concentration optimale des toxoplasmes dans le tampon alcalin est déterminée par référence à un étalon international, et est de 10 000 à 50 000 toxoplasmes par litre.

7. Préparation d'antigènes de toxoplasmes obtenue par un procédé selon l'une quelconque des revendications 1 à 6.

8. Procédé de dosage des anticorps de toxoplasmes dans le sérum, caractérisé en ce qu'on utilise les préparations d'antigènes selon la revendication 7 et en ce que la réaction d'agglutination sur le sérum est conduite en plaques de microtitration en présence de 2-mercapto-éthanol, ou équivalent.

9. Procédé selon la revendication 8, caractérisé en ce que les préparations d'antigènes sont utilisées en l'absence de 2-mercapto-éthanol.

0 040 572

## Patentansprüche

1. Verfahren zur Herstellung eines Toxoplasmaantigenpräparates, bei welchem Mischungen von Toxoplasmen und sarkomatösen Zellen, wie Zellen des TG-180-Sarkoms, Mäusen auf intraperitonealem Weg inokuliert werden und die Toxoplasmen in der erhaltenen Ascitesflüssigkeit gewonnen werden, dadurch gekennzeichnet, daß in zwei Stufen vorgegangen wird: in einer ersten Stufe wird eine Mischung aus einer Toxoplasmen enthaltenden Ascitesflüssigkeit und einer sarkomatöse Zellen enthaltenden Ascitesflüssigkeit Mäusen inokuliert und das peritoneale Exsudat gewonnen, von dem mit den Toxoplasmen infizierte sarkomatöse Zellen durch Zentrifugieren abgetrennt werden; in einer zweiten Stufe wird eine Mischung aus in der ersten Stufe erhaltenen, infizierten sarkomatösen Zellen und aus nicht-infizierten sarkomatösen Zellen Mäusen inokuliert und 48 bis 72 h nach Inokulation wird das peritoneale Exsudat der Mäuse gewonnen, welches der Wirkung von Trypsin während der Zeit unterworfen wird, die gerade notwendig ist, um ein Platzen der Zellen und damit die Freisetzung der Toxoplasmen zu erzielen.

2. Verfahren nach Anspruch 1, dadurch gekennzeichnet, daß in der ersten Stufe Mäusen eine Mischung aus Ascitesflüssigkeit einer zwei oder drei Stunden vorher mit Toxoplasmen inokulierten Maus und Ascitesflüssigkeit von 10 bis 12 Tagen vorher mit sarkomatösen Zellen inokulierten Mäusen inokuliert wird und das Exsudat der mit der Mischung inokulierten Mäuse 48 bis 72 Stunden nach der Inokulation gewonnen wird.

3. Verfahren nach einem der Ansprüche 1 und 2, dadurch gekennzeichnet, daß in der zweiten Stufe das Verhältnis der Anzahl von infizierten sarkomatösen Zellen zu jenem von nicht-infizierten sarkomatösen Zellen je nach Infektionsstadium der Mäuse in der ersten Stufe variiert.

4. Verfahren nach einem der Ansprüche 1 bis 3, dadurch gekennzeichnet, daß das in der zweiten Stufe gewonnene Exsudatsediment in einem Wasserbad bei 37°C der Einwirkung einer Lösung mit 0,05% Trypsin in einer auf einen pH-Wert von 7,2 gepufferten Kochsalzlösung unterworfen wird.

5. Verfahren nach Anspruch 4, dadurch gekennzeichnet, daß die freigesetzten Toxoplasmen nach Sedimentation in einer 6%igen Formaldehydlösung während zumindest 16 Stunden wieder in Suspension gebracht werden, danach zentrifugiert, gewaschen und in einem ein Konservierungsmittel enthaltenden alkalischen Puffer mit einem pH-Wert von 8,7 und in Suspension gebracht werden.

6. Verfahren nach Anspruch 5, dadurch gekennzeichnet, daß die optimale Konzentration der Toxoplasmen im alkalischen Puffer unter Bezugnahme auf einen internationalen Vergleichswert bestimmt wird und zwischen 10 000 und 50 000 Toxoplasmen pro Liter liegt.

7. Toxoplasmaantigenpräparat, hergestellt nach dem Verfahren gemäß einem der Ansprüche 1 bis 6.

8. Verfahren zur Dosierung von Toxoplasmenantikörpern im Serum, dadurch gekennzeichnet, daß die nach Anspruch 7 hergestellten Antigenpräparate verwendet werden und die Agglutinationsreaktion auf das Serum in Mikrotitrationsplatten in Anwesenheit von 2-Mercaptoäthanol od. dgl. durchgeführt wird.

9. Verfahren nach Anspruch 8, dadurch gekennzeichnet, daß die Antigenpräparate in Abwesenheit von 2-Mercaptoäthanol verwendet werden.

## Claims

1. A process for manufacturing a preparation of antigens of toxoplasmas whereby mixtures of toxoplasmas and of sarcomatous cells such as the cells of sarcoma TG 180, are inoculated in mice by the intraperitoneal route, and the toxoplasmas are collected in the ascitic liquid obtained, characterized in that operation is carried out in two steps: in a first step, mice are inoculated with a mixture of ascitic liquid containing toxoplasmas and of ascitic liquid containing sarcomatous cells, and the peritoneal exsudate from which sarcomatous cells infected with toxoplasmas are separated by centrifugation, is collected; in a second step, mice are inoculated with a mixture of infected sarcomatous cells obtained in the first step and of non-infected sarcomatous cells, and from 48 to 72 hours after inoculation, the peritoneal exsudate of the mice is collected and subjected to the action of trypsin for just the time necessary to obtain rupture of the cells, releasing the toxoplasmas.

2. A process according to claim 1, characterized in that in the first step, mice are inoculated with a mixture of ascitic liquid of a mouse inoculated two or three days beforehand by toxoplasmas, and of ascitic liquid of mice inoculated 10 to 12 days beforehand with sarcomatous cells, and the exsudate of the mice inoculated with the mixture is collected from 48 to 72 hours after inoculation.

3. A process according to any one of claims 1 and 2, characterized in that the ratio of the number of infected sarcomatous cells to that of non-infected sarcomatous cells varies as a function of the stage of infection of the mice in the first step.

4. A process according to any one of claims 1 to 3, characterized in that the sediment ot the exsudate collected during the second step is exposed in a water bath at 37° to the action of a 0.05% trypsin solution in a buffered saline solution at pH 7.2.

5. A process according to claim 4, characterized in that the released toxoplasmas are, after sedimentation, replaced in suspension in a 6% solution of formol, for at least 16 hours, then centrifuged, washed and placed in suspension in an alkaline buffer at pH 8.7 containing a preserver agent.

6. A process according to claim 5, characterized in that the optimal concentration of toxoplasmas in the alkaline buffer is determined by reference to an international standard, and is 10 000 to 50 000 toxoplasmas per litre.

7. A preparation of antigens of toxoplasmas obtained with the process according to any one of claims 1 to 6.

8. A process for dosage of the antibodies of toxoplasmas in the serum, characterized in that the preparations of antigens obtained according to claim 7 are used and the reaction of agglutination on the serum is conducted in microtitration plates in the presence of 2-mercaptol-ethanol, or equivalent.

9. A process according to claim 8, characterized in that the preparations of antigens are used in the absence of 2-mercapto-ethanol.